# EUROPEAN PATENT SPECIFICATION

(11) **EP 1 582 218 B1**
(45) Date of publication and mention of the grant of the patent: **29.10.2008**
(21) Application number: 03778232.3
(22) Date of filing: 25.11.2003
(51) Int. Cl.: A61K 39/40, C07K 16/12

(54) **COMPOSITIONS FOR THE TREATMENT OF MYCOBACTERIAL INFECTIONS**
ZUSAMMENSETZUNGFÜR DIE BEHANDLUNG VON MYCOBAKTERIENINFEKTIONEN
COMPOSITIONS DESTINEES AU TRAITEMENT D'INFECTIONS MYCOBACTERIENNES

(30) Priority: 25.11.2002 CU 27502
(43) Date of publication of application: 05.10.2005
(73) Proprietor: Instituto Finlay, Centro de Investigacion-Produccion de vacunas y sueros, La Coronela, La Lisa, Ciudad Habana 11600 (CU)
(72) Inventor: Acosta Dominguez, Armando, Ciudad de la Habana 11300, Cuba (CU); Sarmiento Garcia San Miguel, Maria Elena, Ciudad de la Habana 11300, Cuba (CU); Lopez Hernandez, Yamilé, Ciudad de la Habana 11600, Cuba (CU); Infante Bourzac, Juan Francisco, Ciudad de la Habana 11300, Cuba (CU); Cadiz Lahenz, Armando, Ciudad de la Habana 11500, Cuba (CU); Falero Rodriguez, Gustavo, Playa Ciudad de la Habana 10600, Cuba (CU); Sierra Gonzalez, Victoriano Gustavo, Ciudad de la Habana 16017, Cuba (CU); Martinez Benitez, Máximo Berto, Ciudad de la Habana 11600, Cuba (CU)
(74) Representative: Winckels, Johannes Hubertus F.
(86) International application number: PCT/CU2003/000015
(87) International publication number: WO 2004/047865

(56) References cited:
- US-A1- 2001 007 660
- US-B1- 6 300 104
- ACTOR, J. K, HWANG, S-A., OLSEN, M ET AL: 'Lactoferrin immunomodulation of DTH response in mice' INTERNATIONAL IMMUNOPHARMACOLOGY vol. 2, no. 4, March 2002, pages 475 - 486, XP002992562
- FALERO-DIAZ, G., CHALLACOMBE, S., BANERJEE, D. ET AL: 'Intranasal vaccination of mice against infection with Mycobacterium tuberculosis' VACCINE vol. 18, no. 28, 2000, pages 3223 - 3229, XP004205865
- GLATMAN-FREEDMAN A. ET AL: 'Serum therapy for tuberculosis revisited: Reappraisal of the role of antibody-mediated immunity against Mycobacterium tuberculosis' CLINICAL MICROBIOLOGY REVIEWS vol. 11, July 1998, pages 514 - 532
- TOMODA T. ET AL: '[Tubercle bacilli and the defence factors for infection in sputum and bronchoalveolar lavage fluid]' KEKKAKU vol. 69, no. 12, December 1994, JAPAN, pages 743 - 749

## Description

### TECHNICAL FIELD

The present invention has applications in the field of immunology - specifically in the control of infectious diseases caused by mycobacteria - based on the use of preparations containing antibodies, administered through intra-nasal routes, which have a prophylactic and therapeutic function.

Among existing mycobacterial organisms, we find pathogens which prove dangerous to both animals and man, including *Mycobacterium tuberculosis*, which causes tuberculosis, *Mycobacterium leprae,* responsible for leprosy, *Mycobacterium avium and Mycobacterium intracelulare,* which cause tuberculosis in immunodepressed patients, as well as other mycobacteria which cause diseases in human beings with far less frequency (Sommer HM, Good RC. Mycobacterium. In: Manual of clinical Microbiology, 4th Ed. Washington D.C: A society for Microbiology; 1985. p.216 - 248., Orme IM. Immunity to mycobacteria. Current Opinion in Immunology. 1993; 5: 497 - 502)

In the case of animals, *Mycobacterium avium*, subspecies pararatuberculosis, which causes Jones Disease in ruminants, and *Mycobacterium bovis,* which causes tuberculosis in cattle, are especially noteworthy (Dannenberg Am. Pathogenesis of Tuberculosis: native and acquired resistance in animals and humans. In: Leive L, Schlesinger D (eds). Microbiology. 1984, p. 344 - 354).

Among the most important mycobacterial diseases affecting man, the tuberculosis (TB) infection constitutes a health concern for the entire world and is the primary cause of death associated with infectious diseases, despite the use of the BCG vaccine and of a great number of drugs designed to control it (Dolin PJ, Raviglione MK, Kochi A. Global tuberculosis, incidence and mortality during 1990 - 2000. Bull Who. 2001; 72: 213).

It is estimated that one third of the world's population has been infected by *Mycobacterium tuberculosis.* Every year, 8 million people throughout the world develop active TB and 3 million die from it. A co-infection with the Human Immune-deficiency Virus (HIV), represents 3 to 5 percent of cases (Dolin PJ, Raviglione MK, Kochi A. Global tuberculosis, incidence and mortality during 1990 - 2000. Bull Who. 1994; 72: 213).

The considerable spread of the disease has necessitated the development of new and better diagnostic methods, as well as that of vaccines and therapeutic agents (Collins FM. Tuberculosis: The Return of an Old Enemy. Critical Reviews in Microbiology. 1993; 19: 1 - 16).

With respect to treatment, this has consisted in a combination of medicines administered in relatively high doses and for extended periods of time, something which leads to an associated toxicity that renders difficult the implementation of controlled treatment programs (McCarthy M. Experts see progress in fight against tuberculosis. Lancet. 2002; 359: 2005). In this regard, it would be desirable to shorten the treatment period, to thus favor the application and the completion of a control program, which would prevent the appearance of resistant strains. A reduction in the dosage of the drugs administered would also prove useful in reducing the toxicity of the treatment.

The appearance of strains with multiple resistance to drugs and the great many patients that carry them, as well as the growing number of cases that promise to make an appearance in the future, is a growing concern that demands the development of new therapeutic alternatives (McCarthy M. Experts see progress in fight against tuberculosis. Lancet. 2002; 359: 2005; Hopwell PC. Tuberculosis Control: How the world has changed since 1990. Bull. World Health Org 2002, 80:427, Freire M, Rosigno G. Joining forces to develop weapons against TB: together we must. Bull. World Health Org 2002, 80:429).

In addition to this, there exist many species of mycobacteria that produce diseases in man for which there is no adequate treatment.

The BCG is the only tuberculosis vaccine used in humans. In the entire world, a total of 3 x 10¹² doses have been administered. Its efficacy varies greatly depending on the strain employed the nutritional state, genetic background, age and the presence of intercurrent infections. It is considered efficacious only in the prevention of serious forms of the illness (the miliary and meningeal forms) affecting infants, and to be useless in the prevention of pulmonary tuberculosis, a fact urgently requiring the development of new vaccines (Hirsch LS, Johnson-JL, Ellner JJ. Pulmonary tuberculosis. Curr-Opin-Pulm-Med1999;5(3):143 - 50; Jacobs GG, Johnson JL, Wallis RS. Tuberculosis vaccines: how close to human testing. Tuber Lung Did 1997;78:159 - 169; Ginsberg AM. What's new in tuberculosis vaccines? Bull. World Health Org 2002, 80:483).

The decisive role of cell-mediated immunological responses, fundamentally by the TH1 sub-population, in the fight against *Mycobacterium tuberculosis,* and against mycobacteria in general, has already been recognized and constitutes an international dogma which considers the infection as inevitable (Specificity and Function of T - and B-cell Recognition in Tuberculosis: Pathogenesis, Protection and Control. J infect. Dis.1994; 30: 117 -135).

Following this reasoning, all of the vaccination strategies utilized in the development of new vaccines for use in the fight against tuberculosis and other mycobacteria aim at stimulating this immune response mechanism (Ginsberg AM. What's new in tuberculosis vaccines? Bull. World Health Org 2002, 80:483). However, the role that specific antibodies may play in defense against mycobacteria has not been studied extensively (Glatman A. Casadevall A. Serum therapy for tuberculosis revisited: reappraisal of the role of antibody mediate immunity against Mycobacterium tuberculosis. Clin Microbiol Rev. 1998; 11:514 - 532).

Mycobacterial organisms penetrate the body through the mucosa; this means that the presence of specific antibodies in secretions could constitute a barrier to the penetration of these microorganisms, preventing the infection. Moreover, the arrival of specific antibodies from the mucosa, the blood or other parts of the organism at the site of an existing infection could have therapeutic effects.

To date, the therapeutic work carried out on animals and man involving the use of sera and preparations containing polyclonal antibodies administered by injection, has not yielded conclusive results (Glatman A. Casadevall A. Serum therapy for tuberculosis revisited: reappraisal of the role of antibody mediate immunity against Mycobacterium tuberculosis. Clin Microbiol Rev. 1998; 11: 514 - 532).

In the case of studies conducted on human beings, carried out at the close of the nineteenth century and the beginning of the twentieth, the sera employed were always obtained from other species, with few details given about their form of extraction, their strength, etc., and the investigations carried out lacked the methodological and statistical rigor that would allow for valid conclusions to be reached (Glatman A. Casadevall A. Serum therapy for tuberculosis revisited: reappraisal of the role of antibody mediate immunity against Mycobacterium tuberculosis. Clin Microbiol Rev. 1998; 11: 514 - 532).

Pre-incubation of *Mycobacterium tuberculosis* with a monoclonal antibody, antilipoarabinomannan, before administering the microorganism to mice in aerosol form, prolonged the survival rate in comparison to the group of untreated animals, though it did not reduce the number of microorganisms in the organs studied (Teitelbaum R. Glatman - Freedman A, Chen B, Robbins JB, Unanue E, Casadevall A, Bloom B. A mAb recognizing a surface antigen of Mycobacterium tuberculosis enhances host survival. Proc Natl Acad Sci USA. 1998; 95: 15688 -15693; US Patent Application 20010007660). It is noteworthy that when the effect of administering the antibody "in vivo" through the peritoneum was evaluated, no beneficial effects were detected.

One of the chief disadvantages of the aforementioned strategies is the use of sera and preparations containing antibodies that are obtained from other species, in which antigenic recognition is different, as are the classes and sub-classes of antibodies which - being from species other than man - do not take advantage of all the antibody-dependent, immune effector mechanisms of the human being, greatly reducing their effectiveness. Another disadvantage is found in concomitantly administering heterologous proteins, which leads to serum sickness a serious complication with systemic repercussions, especially for joints and kidneys, together with inactivation of the antibodies due to the response of anti-species antibodies generated by the receptor, leading to the loss of anti-microbial activity in the preparation employed.

In addition to these drawbacks, monoclonal antibodies have the disadvantage of limiting their action to only one epitope of one of the microorganism's antigens, which diminishes their anti-microbial potential and the possibility of employing them against different kinds of mycobacteria. The combination of these elements make these strategies inefficient and dangerous in practice.

The object of the invention presented is the development of preparations containing human antibodies, alone or in combination with immuno-potentiators, immuno-modulators, inactivated or attenuated strains, antigens, genes and/or drugs to be administered through the intra-nasal route, to prevent or treat infections caused by mycobacteria.

The preparations of antibodies employed are made up of:
- human IgG antibodies obtained from plasma, through alcoholic precipitation, ion-interchange chromatography, and a step of viral inactivation.

Optionally, the preparations may comprise
- secretory IgA human antibodies, obtained from colostrum through any of the known, state-of-the-art methods.

All of the preparations were obtained from a large number of individuals, and prior to their employment, the presence of high concentrations of antibodies specific to antigens of *Mycobacterium tuberculosis* and BCG, were detected in the samples. Surprisingly, a wide range of activity against mycobacterial antigens was achieved, allowing for the use of this type of preparation in the prevention and treatment of a broad spectrum of infections produced by this kind of microorganism, bearing in mind the wide, cross reactivity of the antigens among the components of the bacterial species.

These preparations may be used for prophylaxis in groups at risk of Mycobacterium tuberculosis infection, such as those who come in contact with patients (including relatives, friends, professionals, etc.), or among those patients who are especially predisposed to contracting these infections, as is the case of HIV carriers, who are more susceptible to such infections.

On the other hand, these preparations have therapeutic value in the treatment of established infections, such that administering the preparations of antibodies could have a therapeutic effect as well as reducing the treatment period in patients infected with sensitive strains - something of great importance, since the current treatments are lengthy, proving a nuisance to patients and encumbering the work of the public health system; the toxicity of the treatment is increased as is the risk of its not being correctly followed, which can result in the appearance of strains resistant to pharmacological agents, or its application among patients carrying strains with multiple resistance to specific medicines, a growing worldwide phenomenon that currently has no therapeutic alternative.

The preparations representing the present invention produced a significant reduction in levels of BCG pulmonary infections in an intra-nasal model employing mice (example 2). In the preparation of IgG antibodies, a prophylactic and therapeutic effect was also obtained after their administration through the peritoneum. In all of the methods for administering the antibodies, the effective passage of the antibodies to bronchial and salivary secretions has been demonstrated. The inhibition of pulmonary infection was achieved following pre-incubation of the microorganism with the preparations employed (example 2).

The preparations of human gammaglobulin obtained from plasma or colostrum indicated the presence of antibodies that target complete BCG cells and antigens of *Mycobacterium tuberculosis* (example 1); this implies that the resulting immunological effect cannot be linked to antibodies targeting one antigen in particular, and that the simultaneous recognition of numerous different antigens is a positive quality of these types of preparations, stemming from the antibacterial activity of each kind of specific antibody present in the preparation.

Another objective of the invention presented is the use of the preparations mentioned for prophylaxis and treatment of infections in humans caused by any kind of mycobacteria, including those causing tuberculosis, leprosy and others, as well as their administration via intra nasal routes including injection and intra-nasal methods.

The present invention also relates to preparations additionally containing immuno-potentiators and/or immuno-modulators of any kind: natural compounds, recombinant, synthetic, etc., as well as attenuated or inactivated strains, or those containing antigens or their equivalents, or drugs known to be active against mycobacteria.

The present invention offers a novel method for treating and preventing diseases caused by mycobacteria, through the employment of preparations containing IgG and IgA class human antibodies "in vivo". The employment of secretory IgA antibodies is especially novel, being unmentioned by other authors to date. It is equally novel that said preparations proved effective when administered through the mucosa.

The present invention demonstrates, through the results obtained, how human antibodies play a role both in the prevention and in the treatment "in vivo" of a mycobacterial infection, a surprising fact given that antibodies are not described as inhibitors of mycobacterial infections in state-of-the-art techniques, and only cell-mediated protection mechanisms are considered relevant.

The present invention will be described through the following concrete examples:

### Example 1. Distribution kinetics of gammaglobulins

Male mice from an isogenic Balb/c pool, ranging from 8 to 10 weeks in age and from 20 to 22 grams in weight, supplied by the National Centre for the Production of Laboratory Animals (CENPALAB), Havana, Cuba, were employed in the experiment. They were inoculated with the Japanese (Tokyo) BCG strain, produced by the BCG laboratory of Japan, and with preparations of human gammaglobulin, obtained through methods described by Cadiz and co-workers (Cadiz A. Fernandez J. Joo L. Moya A. Modifications to the alcoholic fractioning method employed in Cuba for the production of immunoglobulines for intravenous use. Vaccimotor 1998; 7: 2 -7). As shown in Table 1, the animals in Groups I and II were administered human gammaglobulin (IgG) intra-peritoneously and intra-nasally respectively, and samples of serum, saliva and tracheobronchial secretion were serially extracted from four different animals belonging to each of the groups. A similar experiment was carried out with animals that received the preparation of IgA antibodies intra-nasally

**Table 1. Design of the experiment of human gammaglobulin distribution kinetics in Balb/c mice.**

| Groups | Inoculation Method | Dose of Gammaglobulin | Samples | Extraction Time (hours) |
|---|---|---|---|---|
| I | Intra-peritoneous | 1 mg/g of weight | Serum. Saliva. Tracheobronchial secretion. | 1,2,3 and 5 |
| II | Intra-nasal. (IN) | 1 mg contained in 20 µL | Serum. Saliva. Tracheobronchial secretion | 1,2,3 and 5 |

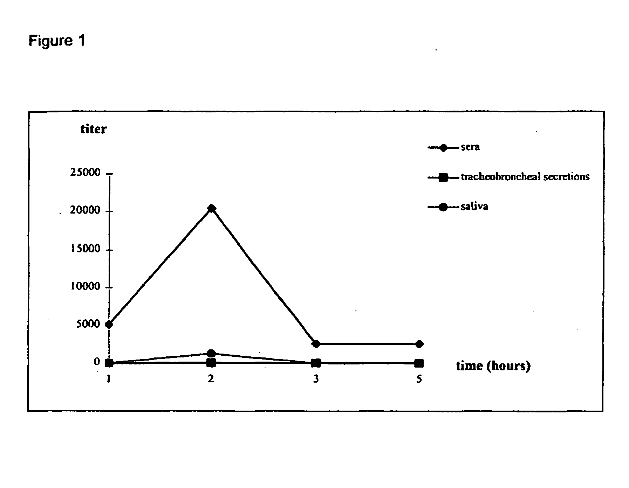

Each sample indicated the presence of antibodies directed against antigens of *Mycobacterium bovis* following an ELISA test of complete cells. Antibodies against BCG surface antigens were detected in the preparation of human gammaglobulin IgG with a titer of 4096. In the case of the preparation containing IgA antibodies, specific antibodies were also detected. In Group I, the samples of serum, extracted in the first period (1 hour), had antibodies with a titer of 5120, which increased to 20480 after 2 hours, decreased to 2560 after 3 hours, and remained stable until the end of the experiment (5 hours). In the case of the saliva samples, the results were similar, with titers of 20, 1280, 0 and 0. The presence of antibodies in the tracheobronchial secretions was not detected until 2 hours had elapsed, with a titer of 80, which decreased to 40 after 3 hours, to reach a value of 20 at the end of the experiment (5 hours). As can be seen, the maximum values for the titers of antibodies corresponding to the samples of serum, saliva and tracheobronchial secretion, were those of 20480, 1280 and 80 respectively. These results are shown in Figure 1.

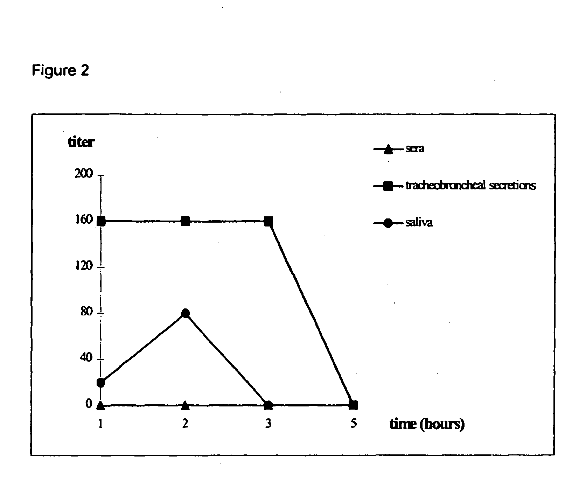

In the animals belonging to Group II that were intra-nasally inoculated with the preparation of IgG gammaglobulin (Figure 2), the presence of specific antibodies was not detected in the blood at the different times of the experiment, though antibodies with a titer of 160 were detected in the tracheobronchial secretion samples that were taken during the first hour of the experiment. Upon comparing the results with respect to the inoculation methods, it was observed that the blood samples indicated the presence of specific antibodies only when the gammaglobulin was inoculated intra-peritoneously. In the saliva samples, greater titer values were observed when the inoculation was done intra-peritoneously, a result related to the high levels of antibodies present in the blood of these animals. In the tracheobronchial secretion, the larger titer values for antibodies were obtained when the inoculation was done intra-nasally. The study demonstrates that with both administration methods, antibodies that recognize the BCG gathered in the mucosa of these animals.
In the case of the preparation of IgA antibodies administered intra-nasally, its pattern of distribution was similar to that obtained with the preparation of IgG administered with the same method.

### Example 2. Challenge experiment

Using the preparations of IgG and IgA secretory antibodies, a challenge experiment was carried out. The design used is shown in Table 2.

**Table 2. Design of the challenge experiment using BCG in Balb/c mice.**

| Groups | Treatment using human gammaglobulin | | Inoculation after two hours | |
|---|---|---|---|---|
| | Dose | Method | Dose | Method |
| I | - | - | BCG, 0.5 x 10⁸ cells | IN |
| II | 1 mg/g of weight | IP | BCG, 0.5 x 10⁶cells | IN |
| III | 1 mg contained in 20 µL. | IN | BCG, 0.5 x 10⁶ cells | IN |
| | | | | |
| IV | - | - | 0.5x10⁸ cells pre-incubated with human gammaglobulin | IN |

The animals in Group I were used as a negative control group and were anesthetized with 0.1 mL of sodium pentobarbital (IMEFA), at 50 mg per kg of weight, through an IV. They were later inoculated intra-nasally with 0.5 x 10⁶ cells of BCG in 50µL of Sauton solution. They were administered 25 µl / nasal cavity using an automatic micro-pipette (Plastomed), following the model for intranasal infection. The mice belonging to Group II were intra-peritoneously treated with human gammaglobulin, at 1 mg per g of weight. Group III mice were intra-nasally inoculated with 1 mg of gammaglobulin contained in 20 µL of solution. After 2 hours, the animals belonging to Groups II and III were "challenged" with 0.5 x10⁸ cells of BCG administered intra-nasally. The animals of Group IV were intra-nasally inoculated with 50 µL of the BCG pre-incubated with human gammaglobulin. To achieve this, 0.5 x 10⁶ cells of BCG were incubated for each mg of human gammaglobulin, lightly agitated at room temperature for a period of 4 hours. The mixture was centrifuged for 10 minutes at 5585 rcf, the precipitate was re-suspended in 500 µL of PBS and was once again centrifuged in the same manner, to finally re-suspend it at a concentration of 10 x 10⁸ cells / mL. All of the animals were then sacrificed, at 24 hours after infection, and their right lungs were extracted in aseptic conditions for mycobiological analysis, consisting in the calculation of the number of colony-forming units per milligram of tissue (CFUs/mg of tissue).

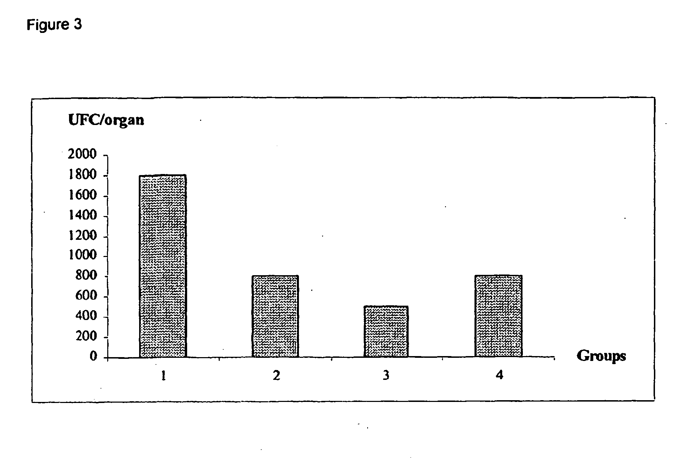

In the case of the preparation containing IgA antibodies, this was intra-nasally administered to a group of animals, following the methodology employed in the case involving the use of the preparation of IgG antibodies, which was also utilized in the pre-incubation of BCG prior to being administered to a group of animals. Group II and III were inoculated intra-peritoneously and intra-nasally respectively with the preparation containing IgG antibodies (Table 2). The dose of gammaglobulin utilized was the same as that used in the kinetics experiment. Following this, the animals were "challenged" with BCG. As we can see in Figure 3, significant differences were encountered (p=0.01) upon comparing the number of CFUs recovered from the animals of Group I (negative control) with those recovered from the remaining groups. No significant differences were found between the number of CFUs recovered from Groups II, III and IV. Similar results were obtained in the group of animals that received the preparation of IgA antibodies intra-nasally and in the group of animals that were inoculated with BCG pre-incubated with this preparation. These results suggest that the interaction of antibodies specific to BCG, following their inoculation or prior to it, exercise a protecting effect which is given by the inhibition or blocking of the entry of microorganisms, by inhibiting their multiplication, as well as by their final elimination through different mechanisms. The challenge experiment demonstrated that both preparations provide similar levels of protection.

### Advantages of the proposed solution

These kinds of preparations have the advantage of being obtained from human beings, sparing us the difficulties related to immunological incompatibility among species (serum sickness, diminished activity of the preparation due to the species' response to it, etc.) and ensuring the presence of antibodies belonging to the same species and all of the subclasses of IgG and IgA, something which guarantees that all of the immunological mechanisms dependent on the antibodies available to the species will be mobilized (neutralization, opsonization, fixing of the complement, ADCC, etc.), resulting in an optimal anti-microbial activity.

Another advantage of this kind of preparation is that it contains IgA secretory antibodies, which ensure that the penetration of the microorganism meets with a blocking or inhibiting activity. This kind of antibody is very stable and has a long half life within secretions due to the presence of the secretory component, making its anti-bacterial action all the more efficient. The presence of human lactoferrin in preparations, which appears together with the IgA secretory antibody following its purification in the colostrum, confers on it an additional anti-microbial activity due to the known, wide-ranging anti-bacterial action of lactoferrin, conditioned by its action over available iron.

Another advantage is the low level of adverse reactions, including the absence of viral transmission by specific periods of viral inactivation. Importantly, these preparations exhibit a prophylactic activity, exercised through a blocking of the microorganism's penetration at the level of the mucosa, and a therapeutic activity exercised through the combined action of different kinds of antibodies, which react with a large number of antigens, unleashing a great variety of anti-microbial mechanisms.

Administering the preparations intra-nasally ensures an uncomplicated and versatile method for introducing the preparations at the site of entry of microorganisms, favoring the inhibition of infection and consequently the prophylactic effect of the preparation, as well as easy access to infected tissues (lung, intestine, etc.), also favoring the therapeutic effect exercised upon established infections.

Another of the advantages considered is the broad spectrum of activity exercised against different kinds of mycobacteria, guaranteed by the high titers of antibodies present and the broad cross reactivity of these antibodies, as well as their extraction from donors with high titer values of antibodies working against mycobacteria in their organism, something which ensures a high level of specific activity.

### Brief description of figures

Figure 1. Distribution kinetics of human gammaglobulin administered intra-peritoneally to Balb/c mice. The values represent the average of the titer values found in the samples extracted at each of the times of the experiment, determined through an ELISA test of complete BCG cells.
Figure 2. Distribution kinetics of human gammaglobulin administered intra-nasally to Balb/c mice. The values represent the average titers found in the samples extracted at each of the times of the experiment, determined through an ELISA test of complete BCG cells
Figure 3. Results of the challenge experiment using Balb/c mice with BCG administered intra-nasally. The values represent the average of the number of CFUs/organ calculated for each group that was studied. The animals from group 1 (positive control) were intra-nasally inoculated with BCG, those belonging to groups 2 and 3 were intra-nasally and intra-peritoneously inoculated, respectively, with human gammaglobulin, before being "challenged" with BCG administered intra-nasally. Group 4 animals were inoculated intra-nasally with BCG, pre-incubated with human gammaglobulin

## Claims

1. Preparation for use in the prevention or treatment of *Mycobacterium tuberculosis* infections in humans, said preparation comprising:
(a) polyclonal human antibodies of type secretory IgA purified from human colostrum, said preparation comprising a high concentration of said antibodies specific to antigens of *Mycobacterium tuberculosis* and BCG,
(b) an appropriate pharmaceutical vehicle, and
(c) optionally a component selected from the group comprising immunomodulators, inactivated or attenuated strains of mycobacterium, lactoferrin, and anti-mycobacterial drugs,
wherein said preparation is for intra-nasal administration.

2. Preparation according to claim 1, wherein said preparation further comprises polyclonal human antibodies of type IgG obtained from human plasma by alcoholic precipitation, ion-exchange chromatography, and a step of viral inactivation.

3. Preparation according to claim 1 or 2, which comprises polygonal human antibodies in a concentration range of 40 to 160 mg/mL

4. Preparation according to any one of claims 1-3, which comprises lactoferrin in a concentration range of 10 to 20 mg/mL.

5. Preparation according to any one of claims 1-4, wherein said immunomodulator is an immunopotentiator.

6. Preparation according to any one of claims 1-5, wherein the immunomodulator is is selected from the group of natural substances, recombinant products, synthetic compounds and genes.

7. Preparation according to any one of claims 1-6, wherein inactivated or attenuated strains of mycobacterium include strains that express heterologous antigens or genes coding for said antigens.

## Patentansprüche

1. Präparat zur Verwendung bei der Prävention oder Behandlung von *Mycobacterium* tuberculosis-Infektionen beim Menschen, wobei das Präparat umfasst:
(a) polyklonale humane Antikörper des Typs sekretorisches IgA, gereinigt aus humanem Colostrum, wobei das Präparat eine hohe Konzentration der Antikörper, die für Antigene von *Mycobacterium tuberculosis* und BCG spezifisch sind, umfasst,
(b) ein geeignetes pharmazeutisches Vehikel und
(c) gegebenenfalls eine Komponente, ausgewählt aus der Gruppe, umfassend Immunomodulatoren, inaktivierte oder attenuierte Stämme von *Mycobacterium,* Lactoferrin und antimykobakterielle Wirkstoffe,
wobei das Präparat zur intranasalen Verabreichung ist.

2. Präparat nach Anspruch 1, wobei das Präparat außerdem polyklonale humane Antikörper des Typs IgG, erhalten aus humanem Plasma durch alkoholische Präzipitation, Ionenaustauschchromatographie und einen Schritt der Virusinaktivierung, umfasst.

3. Präparat nach Anspruch 1 oder 2, das polyklonale humane Antikörper in einem Konzentrationsbereich von 40 bis 160 mg/ml umfasst.

4. Präparat nach einem der Ansprüche 1 bis 3, das Lactoferrin in einem Konzentrationsbereich von 10 bis 20 mg/ml umfasst.

5. Präparat nach einem der Ansprüche 1 bis 4, wobei der Immunomodulator ein Immunopotentiator ist.

6. Präparat nach einem der Ansprüche 1 bis 5, wobei der Immunomodulator ausgewählt ist aus der Gruppe aus natürlichen Substanzen, rekombinanten Produkten, synthetischen Verbindungen und Genen.

7. Präparat nach einem der Ansprüche 1 bis 6, wobei inaktivierte oder attenuierte Stämme von *Mycobacterium* Stämme umfassen, die heterologe Antigene oder Gene, die für die genannten Antigene kodieren, exprimieren.

## Revendications

1. Préparation pour une utilisation dans la prévention ou le traitement des infections de *Mycobacterium tuberculosis* chez l'Homme, ladite préparation comprenant :
(a) des anticorps humains polyclonaux de type IgA sécrétoire purifiés provenant de colostrum humain, ladite préparation comprenant une concentration élevée desdits anticorps spécifiques aux antigènes de *Mycobacterium tuberculosis* et du BCG,
(b) un véhicule pharmaceutique approprié, et
(c) éventuellement un composant choisi dans le groupe comprenant des immunomodulateurs, des souches de mycobactéries inactivées ou atténuées, de la lactoferrine et des médicaments anti-mycobactériens,
dans laquelle ladite préparation est administrée par voie intranasale.

2. Préparation selon la revendication 1, dans laquelle ladite préparation comprend en outre des anticorps humains polyclonaux de type IgG obtenus à partir de plasma humain par précipitation alcoolique, chromatographie par échange d'ions et par une étape d'inactivation virale.

3. Préparation selon la revendication 1 ou 2, qui comprend des anticorps humains polyclonaux dans une gamme de concentrations allant de 40 à 160 mg/mL.

4. Préparation selon l'une quelconque des revendications 1 à 3, qui comprend de la lactoferrine dans une gamme de concentrations allant de 10 à 20 mg/mL.

5. Préparation selon l'une quelconque des revendications 1 à 4, dans laquelle ledit immunomodulateur est un immunopotentiateur.

6. Préparation selon l'une quelconque des revendications 1 à 5, dans laquelle l'immunomodulateur est choisi dans le groupe des substances naturelles, des produits recombinés, des composés synthétiques et des gènes.

7. Préparation selon l'une quelconque des revendications 1 à 6, dans laquelle des souches de mycobactérie inactivées ou atténuées comprennent des souches qui expriment des antigènes hétérologues ou des gènes codant lesdits antigènes.
